# EUROPEAN PATENT SPECIFICATION

(11) **EP 2 963 049 B1**
(45) Date of publication and mention of the grant of the patent: **08.04.2020**
(21) Application number: 14757029.5
(22) Date of filing: 07.02.2014
(51) Int. Cl.: C07D 493/04, C07H 17/04, A61K 36/48, A01N 43/16, A01N 63/02

(54) **METHOD FOR PREPARING COUMESTROL**
VERFAHREN ZUR HERSTELLUNG VON COUMESTROL
PROCÉDÉ DE PRÉPARATION DE COUMESTROL

(30) Priority: 28.02.2013 KR 20130021747
(43) Date of publication of application: 06.01.2016
(73) Proprietor: Amorepacific Corporation, Seoul 140-777 (KR)
(72) Inventor: KANG, Young Gyu, Yongin-si Gyeonggi-do 446-729 (KR); SHIM, Jong Won, Yongin-si Gyeonggi-do 446-729 (KR); SHIM, Jin Sup, Yongin-si Gyeonggi-do 446-729 (KR); PARK, Joon Ho, Yongin-si Gyeonggi-do 446-729 (KR); YEOM, Myeong Hun, Yongin-si Gyeonggi-do 446-729 (KR); CHO, Jun Cheol, Yongin-si Gyeonggi-do 446-729 (KR)
(74) Representative: Novagraaf Technologies
(86) International application number: PCT/KR2014/001040
(87) International publication number: WO 2014/133268

(56) References cited:
- WO-A1-89/07395
- KR-A- 19990 084 092
- KR-A- 20080 104 829
- KR-A- 20130 079 220
- US-A1- 2012 289 714
- US-A1- 2012 322 659
- DURANGO, D. ET AL.: "Phytoalexin Accumulation in Colombian Bean Varieties and Aminosugars as Elicitors", MOLECULES, vol. 7, no. 11, 2002, pages 817-832, XP55296783, DOI: 10.3390/71100817
- LE-VAN, N.: "Coumestrin a coumestan derivative from soybean roots", PHYTOCHEMISTRY, vol. 23, no. 5, 1984, pages 1204-1205, XP55296763, ISSN: 0031-9422, DOI: 10.1016/S0031-9422(00)82649-7
- BOURGAUD, F. ET AL.: 'Production of flavonoids by Psoralea hairy root cultures' PLANT CELL , TISSUE AND ORGAN CULTURE vol. 56, no. 2, 1999, pages 97 - 104, XP055273987
- KORSANGRUANG, SHUNTRA ET AL.: 'Effects of abiotic and biotic elicitors on growth and isoflavonoid accumulation in Puetaria candollei var. candollei and P. candollei var. mirifica cell suspension cultures' PLANT CELL TISSUE AND ORGAN CULTURE vol. 103, no. 3, December 2010, pages 333 - 342, XP019859108

## Description

### [Technical Field]

The present disclosure relates to a method for producing coumestrol.

### [Background Art]

Since the use of inexpensive synthetic estrogens having powerful estrogenic effects such as diethylstilbestrol (DES) was banned in estrogen therapies for menopausal women by the USFDA, natural phytoestrogens that can replace female hormones have garnered research interest. However, because these substances exist in trace amounts in nature, it is very difficult to obtain them in large quantities. Owing to the lukewarm attitude toward large-scale synthesis of such substances over half a century, there has been no remarkable achievement. Although few synthetic methods have been reported, they are only on the laboratory scale and no facile method for large-scale production is currently available. Coumestrol is the most powerful phytoestrogen known until now and is found mainly in the seed, root and leaf of plants in the families Leguminosae and Compositae. It is a type of isoflavonoid and is generally classified as a coumestan-based compound. Coumestrol is secreted at high concentrations at the wound site of the plant and has gained attentions as it is known to prevent infection by bacteria, fungi and viruses through antioxidant, anti-inflammatory and anti-toxin actions (Darbarwar, M., et al., J. Sci. Ind. Res., 35, 297, 1982; Jeffrey, B. H., et al., Phytochemical Dictionary, Taylor & Francis, London, pp 418, 1933). It is because infection by various bacteria, fungi, viruses, etc. induces the synthesis of various aromatic compounds including coumestrol. It is known that the cause of the antibiotic action of coumestrol is the phenolic backbone structure, which inhibits the production of peroxy compounds in vivo by preventing influx of free radical-based oxidizers. In addition, among the various natural coumestan-based derivatives, only coumestrol is known to have estrogenic effect. The estrogenic effect was evaluated by orally administering coumestrol to immature rats and then monitoring the change of uterine weight. It is to be noted that coumestrol exhibited estrogenic effect in young female rats whereas it exhibited no activity in mature male rats and that it exhibited no toxicity at all (Darbarwar, M., et al., J. Sci. Ind. Res., 35, 297, 1982). However, because coumestrol is present in trace amounts in the plants in the family Leguminosae, it is very costly. For this reason, there have been some attempts to obtain coumestrol through synthesis. However, due to its rather complicated chemical structure with several aromatic rings fused, no facile synthesis method has not been reported yet. Although some synthesis methods have been reported, they have many problems and are unsuitable for commercial application. Recently, in the aspect of better safety of naturally-derived substances than those chemically synthesized, methods for large-scale production via natural routes are actively studied.

Document Durango et al., "Phytoalexin Accumulation in Colombian Bean Varieties and Aminosugars as Elicitors", MOLECULES, (2002), vol. 7, no. 11, doi:10.3390/71100817, pages 817 - 832, describes the treatment of bean seedlings with chitosan, this treatment leading to an increased formation of coumestrol.

Document WO89/07395 discloses the treatment of the crop alfalfa with chitosan acetate, but not the production of coumestrol or coumestrin.

Document Bourgaud et al., "Production of flavonoids by Psoralea hairy root cultures", PLANT CELL, TISSUE AND ORGAN CULTURE, (1999), vol. 56, no. 2, pages 97 - 104, discloses the treatment of Lach5 hairy root tissue with high molecular weight chitosan after several days of culture and the subsequent formation of coumestrol.

### [Disclosure]

### [Technical Problem]

A method for producing coumestrol or coumestrin from a plant in the family Leguminosae and producing coumestrol in large quantities through deglycosylation of coumestrin **is disclosed.**

### [Technical Solution]

The present invention as defined by the claims provides a method for producing coumestrol, comprising:
producing coumestrin and coumestrol by treating a plant in the family Leguminosae or soil with chitosan during a vegetative period of the plant in the family Leguminosae; and
deglycosylating the coumestrin by treating the coumestrin with one or more selected from a group consisting of an acid, a base, an enzyme and a microorganism,
wherein the plant in the family Leguminosae is Glycine max,
wherein the chitosan is water-soluble chitosan having a molecular weight of 20,000 or smaller,
wherein the chitosan treatments are performed with 4-6 day intervals. Advantageously, an extract of the plant in the family Leguminosae comprising coumestrin may be treated with one or more selected from a group consisting of an acid, a base, an enzyme and a microorganism.

Advantageously, the extract of the plant in the family Leguminosae may be an extract of a root part of the plant in the family Leguminosae. Advantageously, a root of the plant in the family Leguminosae may be treated with chitosan.

Advantageously, the plant in the family Leguminosae may be treated with chitosan during any of from VE to V4 stages of the vegetative period.

In an aspect, a method for producing coumestrin, including treating a plant in the family Leguminosae or soil with chitosan during the vegetative period of the plant in the family Leguminosae is disclosed.

In another aspect, a method for producing coumestrin, wherein the root of the plant in the family Leguminosae is treated with chitosan is disclosed.

In another aspect, a method for producing coumestrin, wherein the plant in the family Leguminosae is treated with chitosan during any of from VE to V4 stages of the vegetative period is disclosed.

In another aspect, a method for producing coumestrin, wherein low-molecular-weight, water-soluble chitosan is used is disclosed.

In another aspect, a method for producing coumestrin, wherein the chitosan treatment is performed with 4-6 day intervals is disclosed.

In another aspect, a method for producing coumestrin, which further includes preparing an extract of the plant in the family Leguminosae is disclosed.

In another aspect, a method for producing coumestrin, wherein an extract of the root part of the plant in the family Leguminosae is used is disclosed.

In another aspect, a method for producing coumestrol, including treating a plant in the family Leguminosae or soil with chitosan during the vegetative period of the plant in the family Leguminosae is disclosed.

In another aspect, a method for producing coumestrol, wherein the root of the plant in the family Leguminosae is treated with chitosan is disclosed.

In another aspect, a method for producing coumestrol, wherein the plant in the family Leguminosae is treated with chitosan during any of from VE to V4 stages of the vegetative period is disclosed.

In another aspect, a method for producing coumestrol, wherein low-molecular-weight, water-soluble chitosan is used is disclosed.

In another aspect, a method for producing coumestrol, wherein the chitosan treatment is performed with 4-6 day intervals is disclosed.

In another aspect, a method for producing coumestrol, which further includes preparing an extract of the plant in the family Leguminosae is disclosed.

In another aspect, a method for producing coumestrol, wherein an extract of the root part of the plant in the family Leguminosae is used is disclosed.

In another aspect, a method for producing coumestrol, including: producing coumestrin and coumestrol according to the above-described method; and deglycosylating coumestrin by treating the coumestrin with one or more selected from a group consisting of an acid, a base, an enzyme and a microorganism is disclosed.

In another aspect, a method for producing coumestrol, wherein an extract of the plant in the family Leguminosae containing coumestrin is treated with one or more selected from a group consisting of an acid, a base, an enzyme and a microorganism is disclosed.

### [Advantageous Effects]

In an aspect, a method for producing coumestrol or coumestrin, wherein a plant in the family Leguminosae is used, allows for production of coumestrol in large quantities with high yield through increased content of coumestrol or coumestrin produced. The produced coumestrol or coumestrin can be used in various applications, for example, medicine, food or cosmetics.

### [Brief Description of Drawings]

Fig. 1 shows the content of coumestrol in an extract of the root part of a plant in the family Leguminosae depending on inducer treatment and vegetative stages.
Fig. 2 shows a result of conducting an enzymatic reaction using β-glucosidase after dissolving in an extract of the root part of a plant in the family Leguminosae in water and analyzing the precipitate resulting from the enzymatic reaction by HPLC.
Fig. 3 compares the content of coumestrol in an extract of the root part of a plant in the family Leguminosae before and after conducting an enzymatic reaction.

### [Best Mode]

Coumestrol (CMS, 3,9-dihydroxy-6H-benzofuro(3,2-c)(1)benzopyran-6-one C₁₅H₈O₅, MW: 268.2) is a coumarin-like compound which is structurally similar to the natural estrogen estradiol or stilbestrol and has a structure of Chemical Formula 1.

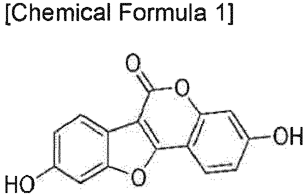

Coumestrin is represented by Chemical Formula 2.

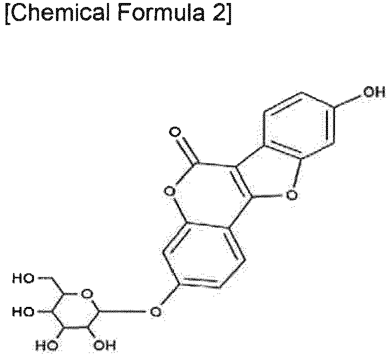

The present disclosure teaches a method for producing coumestrin by treating a plant in the family Leguminosae or soil with chitosan during a predetermined vegetative period of the plant in the family Leguminosae after seeding. The present disclosure also teaches a method for producing coumestrol by treating a plant in the family Leguminosae or soil with chitosan during a predetermined vegetative period of the plant in the family Leguminosae after seeding. Chitosan may be treated to the whole plant of the plant in the family Leguminosae or part of it such as the seed, leaf, stem, fruit, etc. In particular, chitosan may be treated to the root part.

In an aspect of the present disclosure, the plant in the family Leguminosae is not particularly limited as long as it is a plant in the family Leguminosae that produces coumestrin and/or coumestrol (the invention, however, relates to Glycine max only). For example, the plant in the family Leguminosae that can be used in the present disclosure may be one for making bean paste, bean curd or bean sprouts, for boiling with rice, or for use as it is. Breeds for bean paste and bean curd include daepung (Korean Breed Name Registration (hereinafter, omitted) Application Publication No. 2003-940), hojang (Application Publication No. 2003-950), jangwon (Application Publication No. 2002-76), daehwang (Application Publication No. 2000-379), sodam (Application Publication No. 1999-933), songhak (Application Publication No. 1999-936), daewon (Korean National Breed List (hereinafter, omitted) Listing No. 01-0003-1997-38), jinpum (Listing No. 01-0003-1997-26), danbaek (Listing No. 01-0003-1997-18), duyu (Listing No. 01-0003-1997-22), shinpaldal (Listing No. 01-0003-1997-15), taegwang (Listing No. 01-0003-1997-12), manli (Listing No. 01-0003-1997-11), jangsu (Listing No. 01-0003-1997-9), muhan (Listing No. 01-0003-1997-7), baekun (Listing No. 01-0003-1997-2), saeal (Listing No. 10-0003-1997-3), hwanggeum (Listing No. 01-0003-1997-1), jangyeop (Listing No. 01-0003-1997-41), etc. Breeds for bean sprouts include shinhwa-kong (Application Publication No. 2009-982), sowon-kong (Application Publication No. 2000-376), anpyeong (Application Publication No. 2003-939), seonam (Application Publication No. 2003-948), dachae (Application Publication No. 2003-930), sorok, soho (Application Publication No. 2002-78), somyeong (Application Publication No. 1999-932), dawon (Listing No. 01-0003-1997-40), pungsan (Listing No. 01-0003-1997-34), iksan (Listing No. 01-0003-1997-29), sobaek (Listing No. 01-2003-1997-31), gwangan (Listing No. 01-0003-1997-19), danyeop, eunha (Listing No. 01-0003-1997-6), etc. Breeds for boiling with rice include cheongja (Application Publication No. 2002-80), heukcheong (Application Publication No. 2000-381), galmi (Application Publication No. 2000-380), seonheuk (Application Publication No. 1999-934), geomjeong-kong (Listing No. 01-2003-1997-17), ilpum geomjeong-kong (Listing No. 01-0003-1997-42), etc. And, breeds for use as they are include daol (Application Publication No. 2003-931), shinlok (Application Publication No. 2002-77), saeol (Application Publication No. 1999-938), geomjeongol (Listing No. 01-2003-1997-37), seokyang-putkong (Listing No. 01-0003-1997-25), hwaeom-putkong (Listing No. 01-0003-1997-21), keunol (Listing No. 01-2003-1997-14), etc.

In an aspect of the present disclosure, the plant in the family Leguminosae may be treated with chitosan during one or more of VE, VC, V1, V2, V3 and V4 stages of the vegetative period. Specifically, it may be treated with chitosan during from VE to V3 stages, more specifically from VE to V4 stages. The VE stage is a germination stage where seed leaves grow through the soil and the VC stage is a cotyledon stage where primary seedling leaves are developing. The V1 stage is a primary seedling stage where the primary seedling leaves have fully developed, the V2 stage is a first compound leaf stage where first compound leaves have fully developed and the V3 stage is a second compound leaf stage where second compound leaves have fully developed. The V4 stage is a third compound leaf stage where third compound leaves have fully developed.

In addition, the plant in the family Leguminosae and/or soil is treated with chitosan with 4-6 day intervals.

In an aspect of the present disclosure, the chitosan used in the method for producing coumestrol or coumestrin is specifically low-molecular-weight, water-soluble chitosan, more specifically low-molecular-weight, water-soluble chitosan having a molecular weight of 20,000 or smaller, 15,000 or smaller, 10,000 or smaller or 5,000 or smaller. Specifically, the molecular weight of the low-molecular-weight, water-soluble chitosan may be 500 or larger, 1,000 or larger, 2,000 or larger, or 5,000 or larger. The most important property of chitosan is that it is soluble in neutral water while having a small molecular weight. Chitosan is a partially deacetylated chitin and is nontoxic. Although there is no limitation in the concentration of chitosan, it may be specifically 0.01% or higher, more specifically 0.3% or higher. For a solvent used together with chitosan, there is no limitation as long as it dissolves chitosan well and is safe. Specifically, water (H₂O) may be used.

In an aspect of the present disclosure, the method for producing coumestrin may further include a step of preparing an extract of the plant in the family Leguminosae, specifically a step of preparing an extract of the root part of the plant in the family Leguminosae. In another aspect of the present disclosure, the method for producing coumestrol may further include a step of preparing an extract of the plant in the family Leguminosae, specifically a step of preparing an extract of the root part of the plant in the family Leguminosae.

In another aspect of the present disclosure, the method for producing coumestrol may include a step of producing coumestrin and coumestrol by treating a plant in the family Leguminosae or soil with chitosan during a predetermined vegetative period of the plant in the family Leguminosae after seeding and a step of deglycosylating coumestrin by treating the coumestrin with one or more selected from a group consisting of an acid, a base, an enzyme and a microorganism. Coumestrin and coumestrol are produced at the same time by treating the plant in the family Leguminosae or soil with chitosan, and the yield of coumestrol produced can be significantly improved by converting coumestrin to coumestrol through deglycosylation of the coumestrin. That is to say, according to the present disclosure, coumestrol can be produced in large scale as compared to the method of producing coumestrol by treating the plant in the family Leguminosae or soil with chitosan only, by adding the step of deglycosylation of coumestrin.

The extract may be obtained by a method commonly used in the art, such as hot water extraction, high-pressure extraction, reflux extraction, ultrasonic extraction, etc.

Specifically, in order to prepare a plant extract containing coumestrin from the plant in the family Leguminosae using water or an organic solvent, about 1-6 times, specifically about 3 times, of water, one or more organic solvent selected from a group including ethanol, methanol, butanol, ether, ethyl acetate and chloroform or a mixture solvent of the organic solvent and water is added to the plant in the family Leguminosae, e.g., the root of the plant in the family Leguminosae, first. Then, after removing oil by performing extraction at room temperature 1-5 times with stirring, the about 1-8 times, specifically about 4 times, of water or the organic solvent is added to the resulting extract and reflux extraction is performed 1-5 times. Then, after sedimentation at 10-20 °C for 1-3 days, followed by filtration and centrifugation, the filtrate is separated from the residue. The separated filtrate is concentrated under reduced pressure and the resulting extract is suspended in water and pigments are removed using, for example, ether. Then, the aqueous layer is extracted 1-5 times using the organic solvent, and the resulting organic solvent layer is concentrated under reduced pressure to obtain an organic solvent extract, which is dissolved in a small volume of, e.g., methanol. Then, the precipitate produced by adding a large quantity of, e.g., ethyl acetate is dried to obtain the coumestrin-containing extract of the plant in the family Leguminosae of the present disclosure.

Then, coumestrol is prepared from the extract containing the glycoside through hydrolysis using an acid, a base, an enzyme or a microorganism producing the enzyme.

When an acid is used, after adding 0.1-2 N, specifically 1 N, of an acid or a mixture of an acid and an alcohol (specifically a 40-60% ethanol mixture) to the extract, the resulting mixture may be heated at 50-100 °C, specifically in a water bath at 80 °C, for 0.5-2 hours under reflux to obtain a hydrolysis product.

The acid may be one or more acid selected from a group consisting of hydrochloric acid, sulfuric acid and nitric acid, or a mixture of the acid with one or more alcohol selected from a group consisting of ethanol, methanol and butanol.

When a base is used, after dissolving the extract and adding 0.1-2 N, specifically 1 N, of a base or a mixture of a base and an alcohol (specifically a 40-60% butanol mixture), the resulting mixture may be heated at 50-100 °C, specifically in a water bath at 100 °C, for 1-12 hours under reflux to obtain a hydrolysis product.

The base may be one or more base selected from a group consisting of sodium hydroxide and potassium hydroxide, or a mixture of the base with one or more alcohol selected from a group consisting of ethanol, methanol and butanol.

When an enzyme is used, after dissolving the extract in an acidic buffer solution of 5-20 times, specifically about 10 times, and adding an enzyme, the resulting mixture may be heated in a water bath at about 35-40 °C for about 1-60 hours with stirring. After the complete consumption of the substrate is confirmed by thin layer chromatography, the mixture may be heated in hot water (80-100 °C) for 5-15 minutes to terminate the hydrolysis reaction.

When a microorganism producing the enzyme is used, after dissolving the extract in ionized water of 5-10 times, specifically about 10 times, and sterilizing at 110-130°C for 30 minutes, the resulting mixture is cooled to 25-35 °C and then inoculated with a pre-cultured microorganism of 5-10% based on the volume of the mixture. After culturing at 25-35 °C for 2-5 days, followed by centrifugation at 5,000-10,000 rpm, the recovered precipitate is washed several times with distilled water and then centrifuged.

As the enzyme or the microorganism producing the enzyme, an exoglycosidase or a microorganism producing the exoglycosidase may be used. The enzyme can isolate coumestrol by deglycosylating the glycoside.

After performing hydrolysis using the acid, the base, the enzyme or the microorganism producing the enzyme as described above, the reaction solution is concentrated under reduced pressure to remove the solvent. After adding an alcohol to the residue and stirring 1-5 times, the precipitated salts are removed through filtration and the filtrate is concentrated under reduced pressure to obtain a crude product of coumestrol, which is subjected to silica gel column chromatography (e.g., chloroform: methanol = 8:1).

For example, the deglycosylation may be performed as described in Table 1.

**[Table 1]**

| Type | Method |
|---|---|
| Enzyme treatment | After dissolving the extract of the plant in the family Leguminosae in water, enzymatic reaction is conducted using β-glucosidase. |
| Acid treatment | A pH 2-3 solution is prepared using sulfuric acid. After dissolving the extract of the plant in the family Leguminosae in the solution, reaction is conducted at 60-70 °C for 1-4 hours. |
| Microorganism treatment | After dissolving the extract of the plant in the family Leguminosae in ionized water of 5-10 times, the mixture is sterilized at 110-130 °C for 20-40 minutes and then cooled to 20-40 °C. After inoculating with a pre-cultured microorganism of 5-10% based on the volume of the mixture and culturing at 20-40 °C for 2-5 days, followed by centrifugation at 5,000-10,000 rpm, the recovered precipitate is washed 2-4 times with distilled water and then centrifuged. |
| Base treatment | After adding 0.1-2 N of a base or a mixture of a base and an alcohol (specifically a 40-60% butanol mixture) to the extract of the plant in the family Leguminosae, the resulting mixture is hydrolyzed by heating at 50-100 °C for 1-12 hours under reflux. |

The treatment of the substance containing coumestrin with one or more selected from a group consisting of an acid, a base, an enzyme or a microorganism may be conducted on the extract of the plant in the family Leguminosae as it is. Alternatively, the treatment may be conducted after filtering or purifying the extract of the plant in the family Leguminosae to produce coumestrol.

Hereinafter, the present disclosure will be described in detail through experimental examples.

### [Experimental Example 1] Treatment of plant in the family Leguminosae with inducer during vegetative period

A plant in the family Leguminosae (soybean, *Glycine max*) was cultivated while treating the plant in the family Leguminosae and soil with low-molecular-weight, water-soluble chitosan, or *Aspergillus oryzae* **for comparative purposes,** from initial (VE) to V4 stages of the vegetative period. The low-molecular-weight, water-soluble chitosan is suitable for treatment on soil because it dissolves well in water and is unharmful to human when compared with other inducers. As the low-molecular-weight, water-soluble chitosan water-soluble chitosan (YWCTS-100) was purchased from Youngchipharm(KR). The chitosan was dissolved in water to a concentration of 0.3% and sprayed around the stem and soil with 5-day intervals.

*Aspergillus oryzae* is a food-fermenting fungus which is known to have excellent inducing effect through many studies and is unharmful to human. Dried *Aspergillus oryzae* was purchased from Mediogen(KR). One day before the treatment with *Aspergillus oryzae,* 10 g of sugar was dissolved in 1 L of water and 10 g of the dried *Aspergillus oryzae* was mixed well. Then, after incubation in a shaking incubator at 30 °C for 12 hours, the mixture was sprayed around the stem of the bean and soil. The *Aspergillus oryzae* treatment was conducted with 5-day intervals. Purified water of the same volume was sprayed for the control group.

### [Experimental Example 2] Preparation of bean root extract

The root of the beans treated with chitosan and *Aspergillus oryzae* or not treated was harvested at different vegetative stages (6 stages from VE to V4 stages), washed with purified water, dried and then pulverized. The resulting bean root powder (100 g) was added to 1 L of a 70% ethanol aqueous solution and extracted at room temperature for 24 hours. After filtering through 300-mesh filter cloth, the filtrate was condensed under reduced pressure at 50 °C using a distillatory apparatus equipped with a condenser and then dried to obtain a bean root extract.

### [Experimental Example 3] Analysis of coumestrol content in bean root extract

The coumestrol content of the bean root extract obtained at different vegetative stages in Experimental Example 2 was quantitatively analyzed by HPLC. For comparison of the coumestrol content in the bean root extract, coumestrol purchased from Indofine Chemical was used as standard material.

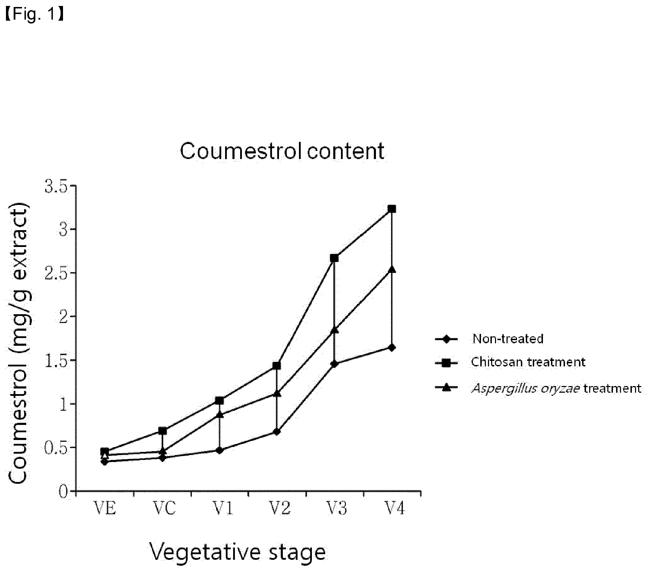

The coumestrol content in the bean root extract depending on inducer treatment and vegetative stages is shown in Fig. 1. It can be seen that the coumestrol content in the bean root extract treated with the inducer increased significantly as compared to the non-inducer-treated control group. In particular, the coumestrol content was increased rapidly in the V3 and V4 stages of the vegetative period. Up to the V4 stage, the content of produced coumestrol was 1.6 mg/g extract for the inducer-treated control group, which was increased about 1.6 times to 2.6 mg/g when treated with *Aspergillus oryzae* and about 2 times to about 3.2 mg/g when treated with chitosan.

### [Experimental Example 4] Increase of coumestrol content through deglycosylation of coumestrin

From Experimental Example 1, it was confirmed that coumestrin, which is a coumestrol glycoside, was produced in the chitosan-treated bean root. The coumestrol content in the extract can be further increased by deglycosylating the coumestrin in the chitosan-treated bean root extract.

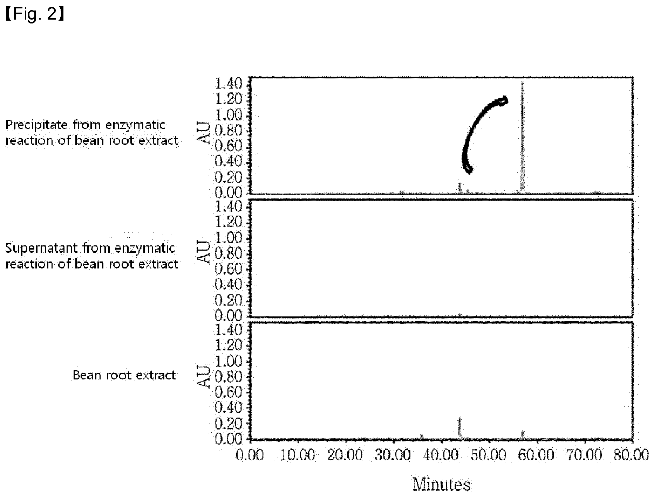

For deglycosylation, an enzymatic reaction was conducted using β-glucosidase and the precipitate produced from the enzymatic reaction was analyzed by HPLC. As seen from Fig. 2, it was found out that the coumestrol content in the bean root extract was remarkably increased as the coumestrin in the extract was converted to coumestrol through deglycosylation. Specifically, the coumestrol HPLC peak around 57.00 minutes was increased by about 15 times in the bean root extract enzymatic reaction precipitate as compared to the bean root extract, whereas the coumestrin peak around 44.00 minutes was slightly decreased.

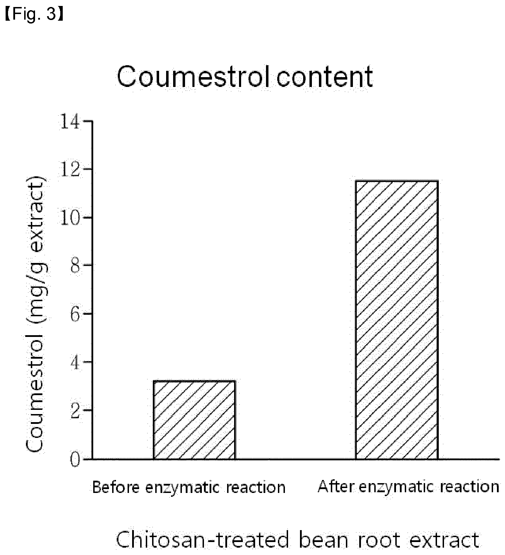

The coumestrol content in the bean root extract before and after the enzymatic reaction is compared in Fig. 3. As a result of the enzymatic reaction, the coumestrol content in the bean root extract was increased by about 3.5 times through the chitosan treatment.

## Claims

1. A method for producing coumestrol, comprising:
producing coumestrin and coumestrol by treating a plant in the family Leguminosae or soil with chitosan during a vegetative period of the plant in the family Leguminosae; and
deglycosylating the coumestrin by treating the coumestrin with one or more selected from a group consisting of an acid, a base, an enzyme and a microorganism,
wherein the plant in the family Leguminosae is Glycine max,
wherein the chitosan is water-soluble chitosan having a molecular weight of 20,000 or smaller,
wherein the chitosan treatments are performed with 4-6 day intervals.

2. The method according to claim 1, wherein an extract of the plant in the family Leguminosae comprising coumestrin is treated with one or more selected from a group consisting of an acid, a base, an enzyme and a microorganism.

3. The method according to claim 2, wherein the extract of the plant in the family Leguminosae is an extract of a root part of the plant in the family Leguminosae.

4. The method according to claim 1, wherein a root of the plant in the family Leguminosae is treated with chitosan.

5. The method according to claim 1, wherein the plant in the family Leguminosae is treated with chitosan during any of from VE to V4 stages of the vegetative period.

## Patentansprüche

1. Verfahren zur Herstellung von Coumestrol, bei dem man:
Coumestrin und Coumestrol herstellt, indem man eine Pflanze aus der Familie der Leguminosae oder Erdboden während einer vegetativen Periode der Pflanze aus der Familie der Leguminosae mit Chitosan behandelt, und
das Coumestrin deglykosyliert, indem man das Coumestrin mit einem oder mehreren ausgewählt aus einer aus einer Säure, einer Base, einem Enzym und einem Mikroorganismus bestehenden Gruppe behandelt,
wobei es sich bei der Pflanze aus der Familie der Leguminosae um Glycine max handelt,
wobei es sich bei dem Chitosan um wasserlösliches Chitosan mit einem Molekulargewicht von 20 000 oder weniger handelt,
wobei die Chitosanbehandlungen in Abständen von 4-6 Tagen erfolgen.

2. Verfahren nach Anspruch 1, bei dem man einen Coumestrin umfassenden Extrakt der Pflanze aus der Familie der Leguminosae mit einem oder mehreren ausgewählt aus einer aus einer Säure, einer Base, einem Enzym und einem Mikroorganismus bestehenden Gruppe behandelt.

3. Verfahren nach Anspruch 2, wobei es sich bei dem Extrakt der Pflanze aus der Familie der Leguminosae um einen Extrakt eines Wurzelteils der Pflanze aus der Familie der Leguminosae handelt.

4. Verfahren nach Anspruch 1, bei dem man eine Wurzel der Pflanze aus der Familie der Leguminosae mit Chitosan behandelt.

5. Verfahren nach Anspruch 1, bei dem man die Pflanze aus der Familie der Leguminosae während eines der VE- bis V4-Stadien der vegetativen Periode mit Chitosan behandelt.

## Revendications

1. Procédé de production de coumestrol, comprenant :
la production de coumestrine et de coumestrol en traitant une plante de la famille des Leguminosae ou un sol avec du chitosane pendant une période végétative de la plante de la famille des Leguminosae
la déglycosylation de la coumestrine par traitement de la coumestrine avec un ou plusieurs éléments choisis dans un groupe constitué d'un acide, d'une base, d'une enzyme et d'un micro-organisme,
où la plante de la famille des Leguminosae est la Glycine max,
où le chitosane est un chitosane soluble dans l'eau ayant un poids moléculaire de 20 000 ou moins,
où les traitements au chitosan sont effectués à des intervalles de 4 à 6 jours.

2. Méthode selon la revendication 1, dans laquelle un extrait de la plante de la famille des Leguminosae comprenant de la coumestrine est traité avec un ou plusieurs éléments choisis dans un groupe constitué par un acide, une base, une enzyme et un micro-organisme.

3. Méthode selon la revendication 2, dans laquelle l'extrait de la plante de la famille des Leguminosae est un extrait d'une partie de racine de la plante de la famille des Leguminosae.

4. Méthode selon la revendication 1, dans laquelle une racine de la plante de la famille des Leguminosae est traitée avec du chitosan.

5. The method according to claim 1, wherein the plant in the family Leguminosae is treated with chitosan during any of from VE to V4 stages of the vegetative period.
